# EUROPEAN PATENT SPECIFICATION

(11) **EP 1 884 213 B1**
(45) Date of publication and mention of the grant of the patent: **17.03.2010**
(21) Application number: 07252585.0
(22) Date of filing: 26.06.2007
(51) Int. Cl.: A61B 17/70, A61B 17/80

(54) **A bone plate**
Knochenplatte
Lame osseuse

(30) Priority: 28.06.2006 US 476344
(43) Date of publication of application: 06.02.2008
(73) Proprietor: DePuy Products, Inc., Warsaw, IN 46581 (US)
(72) Inventor: Long, Jack F., Warsaw, IN 46582 (US); Auger, Daniel D., Ft. Wayne (US); Steinberger, Douglas J., Fremont, OH 43420 (US)
(74) Representative: Gover, Richard Paul

(56) References cited:
- WO-A-2004/086990
- WO-A-2006/028971
- US-A1- 2006 009 770

## Description

This application relates generally to the field of orthopaedics, and more specifically to bone plates and systems for stabilization and compression of a fractured or otherwise damaged bones.

Osteosynthesis is a surgical procedure that stabilizes and joins the ends of fractured bones by mechanical devices such as metal plates, pins, rods, wires or screws. Bone plates are commonly used in osteosynthesis. A Bone plate generally comprises a bone contacting surface, and upper surface opposite the bone contacting surface, and a plurality of holes extending through the plate. The holes in a bone plate are configured to receive bone screws that secure the plate to the bone.

In general two different types of bone plates have been available in the past. A first type of plate is the compression plate. The holes formed in a compression plate include compression ramps formed along the sides of the holes. These compression ramps are designed to engage rounded screw heads on bone screws. When the bone screw is driven into a bone and the screw head engages the compression ramp, the bone plate is moved relative to the screw by the force of the screw head acting against the compression ramp. By strategically placing bone screws in the holes of a compression plate, the bone plate may be used to compress opposite sides of a bone toward a fracture. Such compression may help facilitate healing with certain types of fractures. Compression plates may also be used to pull fractured bones into better alignment, so as to position the bone better for healing. In addition to the above, the screw head forces the bone plate tightly against the bone, and this compression is advantageous in the healing of certain fractures, such as compound fractures.

A second type of bone plate is the fixation or locking plate. The holes formed in locking plates include threads designed to engage complimentary threads formed in the head of a bone screw. When the threads in the holes of the bone plate engage the threads in the head of a bone screw, the bone plate is locked in place relative to the bone screw. Locking plates are useful in maintaining the proper bone length and for fixing the bone ends in their proper anatomic alignment during healing. With a locking plate, the bone plate is generally not compressed against the bone or fracture, and consequently the blood flow in this area is not inhibited, resulting in faster healing for certain types of fractures.

As mentioned above, different types of bone plates may be used for different reasons. However, in some situations, a surgeon may wish to make simultaneous use of advantages offered by the different plates. Accordingly, some prior art bone plates have included different types of holes. In particular, some prior art bone plates have included a first set of holes characteristic of a compression plate, and a second set of holes characteristic of a locking plate. Of course, when two sets of holes are provided the overall number of holes in the plate increases and this may weaken the structural integrity of the plate. Moreover, for relatively small plates, it may be difficult to provide the two different types of holes in the correct location on the plate. Furthermore, when two types of holes are present, the surgeon may mistake a hole of one type for a hole of a different type, resulting in an improperly positioned screw or hole in the bone.

WO-2004/086990 discloses a locking element and a corresponding housing for locking of the locking element in said housing. The locking element comprises a locking piece, for example a head, with a peripheral outer surface running essentially along the direction of a longitudinal axis with at least one clamping surface extending outwards from the longitudinal axis in the form of a wedge in order to lock the locking piece on the locking element with a corresponding inner contour of the housing.

WO-2006/028971 discloses a translational bone fixation assembly. The bone fixation assembly may be used in spinal fusion procedures in which a damaged or diseased disc is removed from between a pair of vertebrae and a spinal fusion spacer is placed between the vertebrae. The bone fixation assembly may be applied to an anterior portion of the affected vertebrae to span the affected disc space and may be fixed to the vertebrae using bone screws. Holes formed within the fixation assembly may incorporate a retention clip arranged to engage a groove formed within a head portion of a bone screw.

The present invention provides a bone plate which has a bone facing surface, an outward facing surface opposite the bone facing surface, and at least one hole extending through the bone plate from the outward facing surface to the bone facing surface. An arced compression surface is provided within the at least one hole and a cam path comprising a cam groove forms an indentation in the arced compression surface.

The bone plate of the invention has holes which can be used in a compression fashion or a locking fashion. The holes can be used in a manner which provides the functionality of holes dedicated to either compression or locking. The holes can be used in a manner which can provide a combination of both compression and locking features.

The holes in the bone plate may be provided in various shapes and sizes. For example holes can be provided which are elliptical, or which are circular. The arced compression surface generally extends 360° within the at least one hole. However, the arced compression surface may be broken up such that it extends less than 360° around the hole.

The cam path generally extends one revolution or less around the hole upon the arced compression surface. Furthermore, a single or multiple cam paths may be provided along each arced compression surface. The cam path can be helical in shape, starting at an upper edge of the arced compression surface. The cam path can be flat with respect to the bone facing surface of the bone plate, positioned at a lower edge of the arced compression surface.

The bone plate can be configured for use with either a compression bone screw or a locking bone screw. The compression bone screw includes a head with a cupped lower surface that does not include a cam. When the compression screw is used with the bone plate, the screw head engages the compression ramps provided in the holes of the bone plate, allowing the bone plate to be used for compression applications.

The locking screw also includes a head with a cupped lower surface, but a cam is provided on the cupped lower surface. The cam protrudes from the arced compression surface and extends one revolution or less around the cupped lower surface of the bone screw. The cam may be provided on the head of the screw in a relatively flat manner or in a helical fashion. In one embodiment, the cam is releasibly connected to the head. In this embodiment, the cam is C-shaped and the head comprises a cam groove configured to receive the C-shaped cam. When the locking screw is used with the bone plate, the cam of the locking screw engages the cam path provided in the hole of the bone plate, thus locking the bone screw to the bone plate.

The bone plate of the invention can be used in a method of securing a bone plate to a bone, the method comprising:
a) providing a bone plate including a hole configured to receive either a compression screw or a locking screw, in which the hole includes a locking structure and a compression structure, and in which both the compression screw and the locking screw include a screw head and a screw shank;
b) selecting either the compression screw or the locking screw; and
c) inserting the selected screw into the hole with the screw shank passing through the hole and into the bone with the screw head engaging the hole.

Embodiments of the invention will now be described by way of example with reference to the accompanying drawings, in which:
FIG. 1 shows perspective view of a bone plate with a plurality of holes and bone screws extending through the holes, in which the holes include both compression features and locking features for the bone plate;
FIG. 2 shows a perspective view of an alternative embodiment of the bone plate and bone screws of FIG. 1;
FIG. 3 shows a perspective view of another alternative embodiment of the bone plate and bone screws of FIG. 1;
FIG. 4A shows a perspective view of an embodiment of a hole for the bone plate of FIG. 1;
FIG. 4B shows a cross-sectional view of the bone plate and hole of FIG. 4A;
FIG. 5A shows a perspective view of an alternative embodiment of a hole for the bone plate of FIG. 1;
FIG. 5B shows a cross-sectional view of the bone plate and hole of FIG. 5A;
FIGS. 6A-6C show three perspective views of a bone screw being inserted into the hole of FIG. 4A in a locking fashion;
FIGs. 7A-7B show alternative positions for engagement of a bone screw with the hole of FIG. 4A;
FIGs. 8A-8C show three perspective views of a bone screw being inserted into the hole of FIG. 4A in a compression fashion;
FIG. 9A shows an alternative embodiment of a bone screw having a detachable cam;
FIG. 9B shows the detachable cam positioned on the bone screw of FIG. 9A;
FIGS. 10A-10C show three perspective views of the bone screw of FIG. 9C being inserted into the hole of FIG. 5A using rotation of the detachable cam to lock the cam to the bone plate;
FIG. 11 shows a tool configured to rotate the detachable cam of FIG. 9A within a bone plate; and
FIGS. 12A-12C show three perspective views of the bone screw of FIG. 9C being inserted into the hole of FIG. 5A in a self-locking manner.

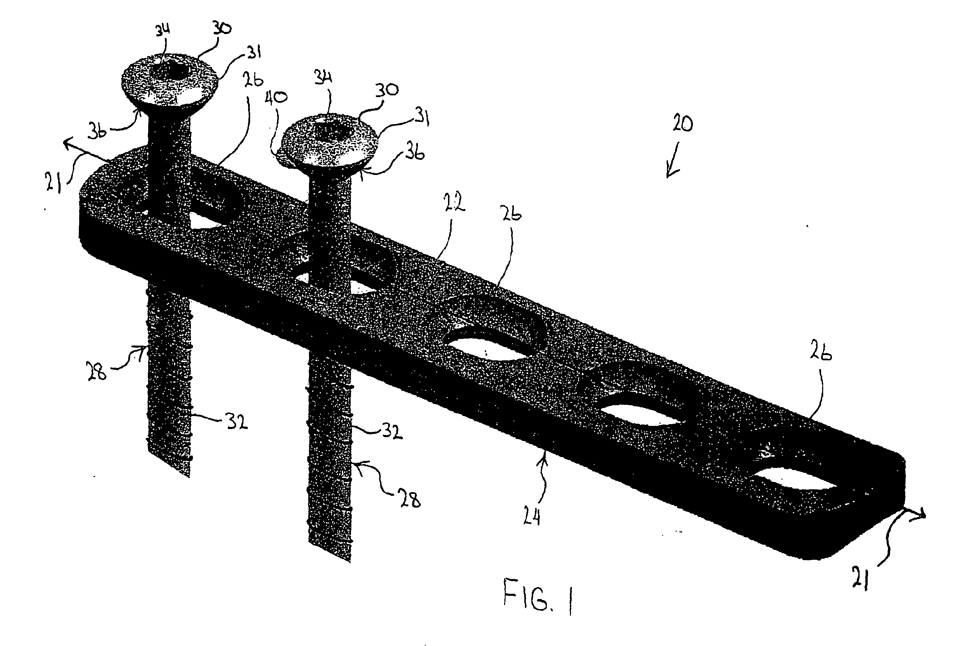

Referring to the drawings, FIG. 1 shows a cam/compression bone plate 20 comprises an outward facing surface 22 and a bone facing surface 24 opposite the outward facing surface 22. The bone plate may be elongated in shape such that it defines a central longitudinal axis 21 extending across the length of the elongated plate. A plurality of holes 26 extend through the plate from the outward facing surface 22 to the bone facing surface 24. The holes 26 are generally provided along the longitudinal axis 21, but may also be positioned elsewhere on the plate 20. Each of the plurality of holes is configured to receive a bone screw 28. Both the bone plate 20 and the bone screws 28 are comprised of bio-compatible materials. Examples of such bio-compatible materials include titanium, nickel, cobalt chromium, and other bio-compatible materials as will be recognized by those of skill in the art.

### The Bone Screw

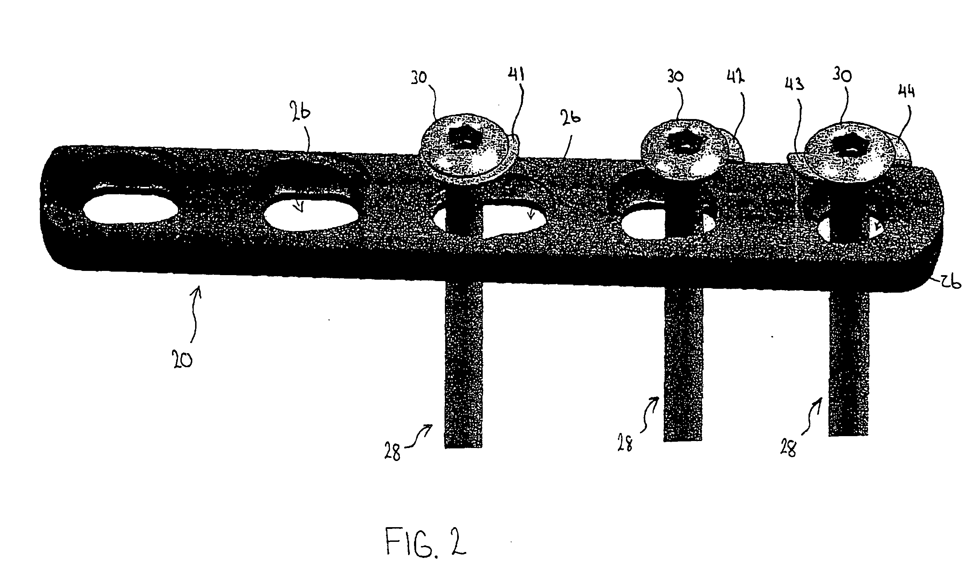

As shown in FIGs. 1 and 2, each bone screw 28 generally comprises a head 30 with a threaded shaft 32 extending from the head 30. The screw head 30 includes a generally circular perimeter portion 31. A slot 34 is formed in the top of the screw head. The slot 34 is configured to receive the tip of a screw driving mechanism, thus allowing the screw 28 to be rotated and the threaded shaft 32 driven into the bone. The lower portion of the screw head 30 comprises a cupped surface 36. As will be recognized by those of skill in the art, the cupped surface 36 of the screw head 30 is configured to engage a compression surface on the bone plate.

In one embodiment of a bone screw 28 configured for use with the bone plate 20 described herein, at least one cam member 40 is provided on the screw head 30. The cam member 40 is generally provided as a crescent-shaped protrusion on the cupped lower surface 36 of the screw head, just below the perimeter 31 of the head 30. However, the cam member 40 may take any number of different forms and shapes. For example, the cam member 40 may comprise a small curved protrusion spanning less than thirty degrees around the cupped surface 36, as shown in FIG. 1.

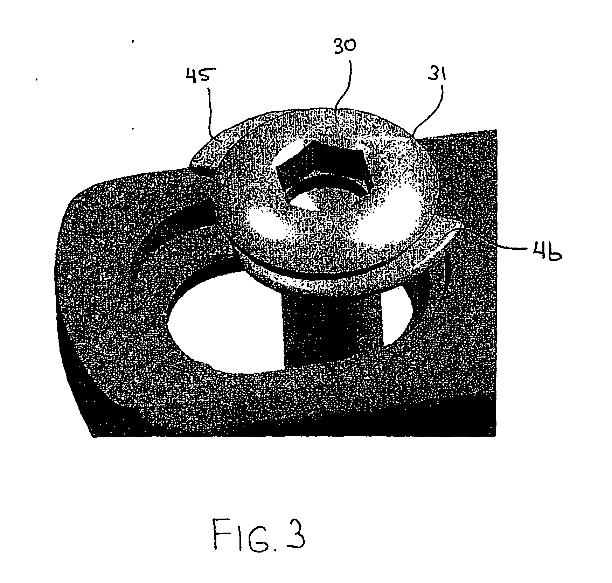

Several other embodiments of a cam member positioned on the head of a bone screw are shown in FIGS. 2 and 3. In one embodiment shown in FIG. 2, the cam member 41 extends more than 90 degrees, but less than 360 degrees around the cupped surface of the bone screw. In another embodiment shown in FIG. 2, the cam member 42 is larger than the cam of FIG. 1, and protrudes farther from the lower cupped surface of the head. In addition, the cam member 42 extends between 45 degrees and 90 degrees around the head 30. In yet another embodiment shown in FIG. 2, the head 30 of the bone screw comprises two cam members 43, 44. The two cam members 43, 44 are directly opposed to one another on the cam head 30. In this embodiment, the two cam members 43, 44 each extend approximately forty-five to ninety degrees around the head 30.

Another embodiment where the head 30 includes two cams is shown in FIG. 3. In this embodiment, the cams 45, 46 are directly opposed to each other, with each cam extending between ninety and one hundred eighty degrees around the screw head 30. In this and other embodiments, each cam 45, 46 may extend around the head 30 in a helical fashion or may be level and parallel with perimeter 31 of the screw head. In any event, the exact shape, size, and configuration of the cam 40 will depend on the type of hole and associated cam slot that the bone screw 28 is configured for use with.

### Bone Plate Holes

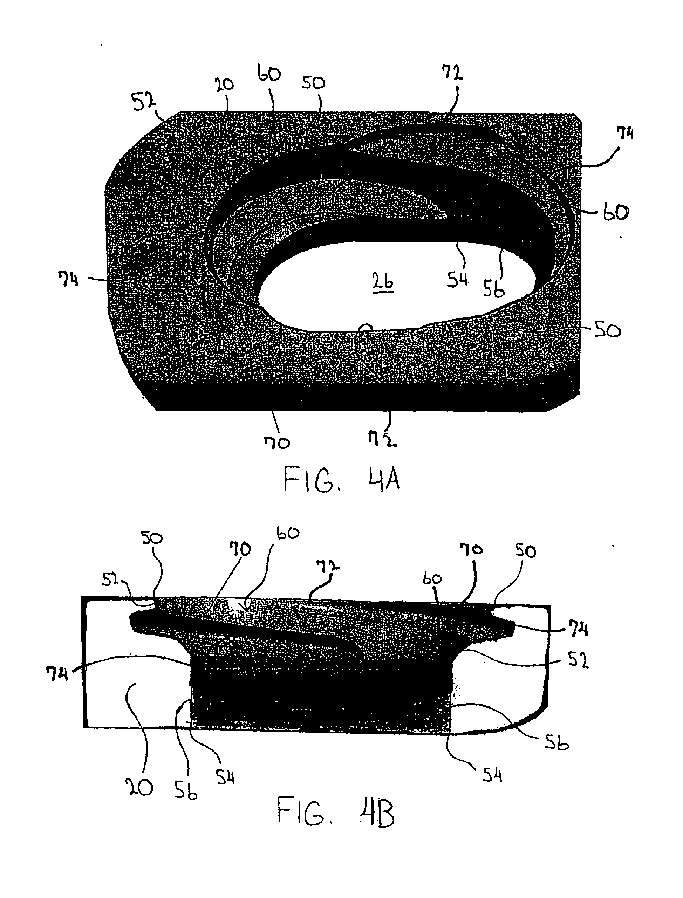

The configuration of holes in the bone plate 20 and associated cam slots are now described with reference to FIGs. 4A and 4B. Each hole 26 includes an upper edge 50 and a lower edge 54. The upper edge 50 begins an upper portion 52 of the hole 26, and the lower edge 54 ends a lower portion 56 of the hole 26. A compression surface 60 (also referred to herein as a "compression ramp") is provided in the upper portion 52 of the hole and generally encircles the upper portion of the hole.

The compression surface 60 is cup-shaped, resulting in an arced compression surface. In other words, if a cross-section of the hole 26 is taken along the plane parallel to the axis of insertion for the bone screw, the compression surface will generally appear as a curved line along the side of the hole, and such line generally moves toward the centre of the hole from top to bottom (for example as shown in FIG. 4B). Accordingly, the compression surface 60 is configured to engage a screw head 30, and particularly the cupped lower surface 36 of the screw head. As will be recognized by those of skill in the art, the engagement of the compression surface 60 of the bone plate 20 and the cupped surface 36 on the head of a bone screw 28 is operable to provide compression to a fractured bone. In addition, the compression surface 60 is provided on at least two directly opposite sides of the hole 26 along the central axis 21, or an axis parallel thereto. This allows the plate 20 to provide compression in one of at least two directly opposite directions. In the disclosed embodiment, the compression surface 60 substantially surrounds the hole 20, allowing the compression plate to provide compression in any of numerous directions parallel to the plane of the plate.

With continued reference to FIGs. 4A and 4B, it can be seen that at least one cam path 70 is formed in the hole 26. The at least one cam path 70 is cut into the compression surface 60, disrupting the continuity of the compression surface 60. The at least one cam path 70 includes a mouth 72 formed in or near the upper edge 50 of the hole 26. The mouth 72 of the cam path 70 feeds into a groove 74 that winds around the compression surface 60 in a helical fashion. The groove 72 generally extends less than one complete turn (i.e., less than 360 degrees) around the compression surface 60. The cam path 70 is configured to receive the cam provided on the head of the bone screw 28.

As shown in FIG. 4A, the hole 26 may include more than one cam path 70. Two cam paths 70 are provided in the embodiment of FIG. 4A. The mouths 72 of the two cam paths are provided directly opposite each other on the upper edge 50 of the hole 26. The grooves 74 extending from these mouths 72 do not intersect, but wrap around the compression surface 60 in a helical fashion. While the total span of the combined grooves may be more than 360° within the hole 26, no single groove 74 wraps around the compression surface more than 360°. In the embodiments providing more than one cam path 70, either cam path may be selected to lock the screw to the plate 20. Furthermore, in some embodiments where a screw 26 with multiple cams is used (e.g., see FIG. 3), each cam on the screw head may engage one of the cam paths 70 on the bone plate 20. In yet other embodiment, either cam on a screw head may engage one of the cam paths on the bone plate.

The hole 26 on the bone plate 20 may be provided in various sizes. In FIGs. 4A and 4B the hole 26 is generally elliptical in shape with two cam paths 70 provided within the hole 26. However, one of skill in the art will recognize that the hole itself may be configured in various shapes and sizes. For example, in FIG. 1 the holes 26 are elliptical in shape but include only a single cam path. In FIG. 2, holes 26 of differing sizes are shown, including a round hole with two cam paths. In another embodiment, a circular hole may be even larger in order to allow for compression by a screw in one of several directions, including laterally or longitudinally and/or increments in between.

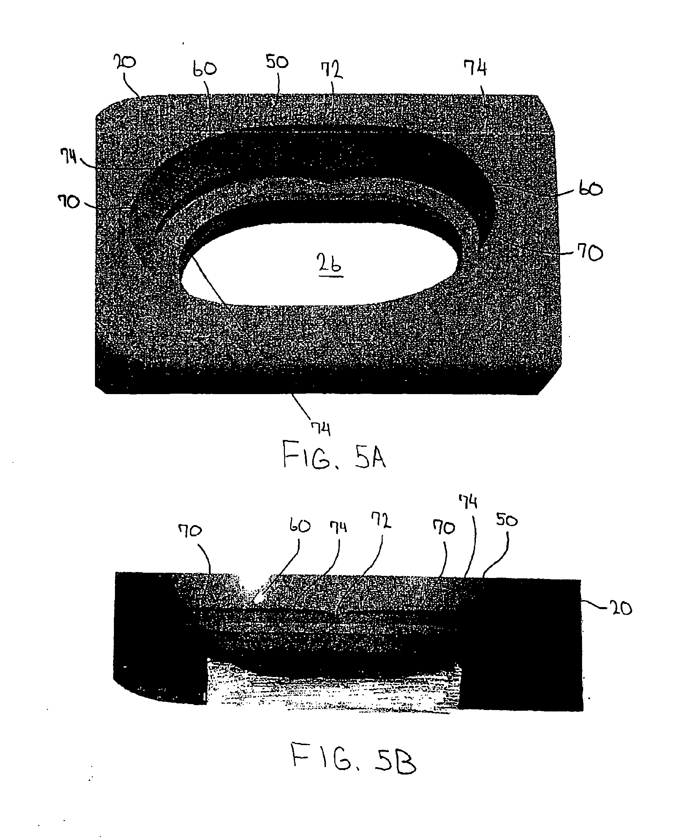

Another alternative embodiment of a hole configured for use with the cam/compression plate 20 is shown in FIGS. 5A and 5B. In this embodiment, two opposing cam paths 70 are provided in the hole 26, but the cam paths 70 are flat instead of helical. Accordingly, the cam paths 70 are parallel with the upper surface 22 of the plate 20. The cam paths 70 are provided at the lower end of the compression surface 60, and do not otherwise cut into the compression surface 60. Each cam path includes a groove 74 of constant size that extends about 180° around the hole 26. A small mouth 72 that leads to the groove is provided where the opposing cam paths meet. Because the cam paths 70 of FIGs. 5A and 5B are flat, the cam paths are only configured to engage small cams or cams of parallel non-helical configuration provided on a screw head. In addition, the flat configuration of these cam paths may facilitate a cam being locked in place when the screw head moves a sufficient distance within the hole such that the cam snaps into the cam path. One of skill in the art will recognize that numerous other variations cam paths are possible in addition to those shown in FIGs. 4A to 5B.

### Locking Between Bone Screw and Bone Plate

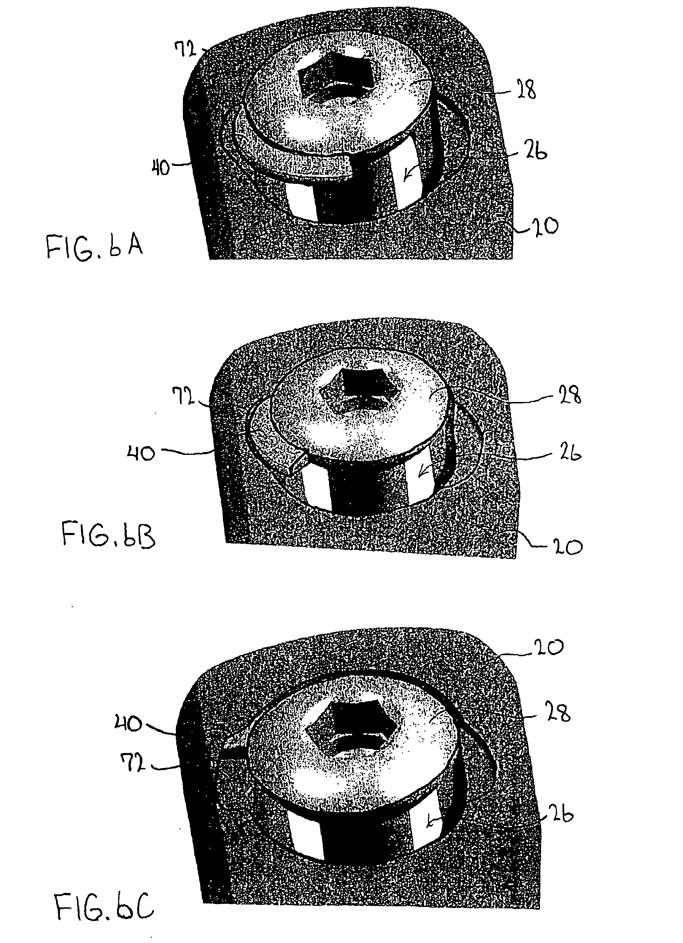

FIGs. 6A to 6C show a bone screw 28 being locked to a bone plate 20. In FIG. 6A, the cam 40 is shown resting at the mouth 72 of the cam path on the bone plate 20. After the cam 40 enters the mouth 72 of the cam path, the cam 40 follows the groove 74 as the bone screw 28 continues to rotate. In FIG. 6B, the bone screw 28 has been rotated such that the cam 40 is engaging the groove of the cam path and the rear portion of the cam 40 is entering the cam mouth 72. Then, in FIG. 6C, the bone screw 28 has been further rotated such that the cam 40 is fully engaging the cam path and has passed through the cam mouth 72. As the cam of the bone screw 28 reaches the end portion of the cam path 70, the head 30 of the bone screw is locked to the bone plate 20. Depending upon the size and shape of the cam, and the associated cam path, locking between the screw head 30 and the plate 20 may be achieved over a relatively short rotational distance or a longer rotational distance. For example, cam paths configured to engage a relatively thick cam that extends a relatively short distance across the head of the bone screw (e.g., 10°) will typically lock much faster than longer cams with a gradual taper that extend a relatively long distance across the head of the screw (e.g., 180°).

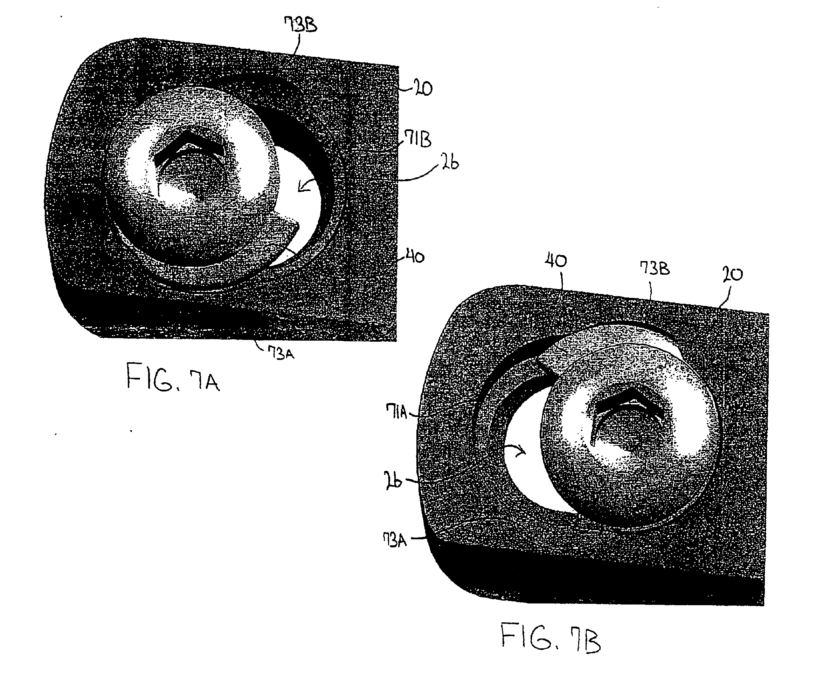

FIGS. 7A and 7B show an embodiment where the hole 26 includes two cam paths, 71 A and 71B. Accordingly, two mouths 73A and 73B are shown at the start of the cam paths 71 A, 7 1 B. In FIG. 7A, the bone screw 28 has been placed in the hole 26 such that the cam 40 will enter the mouth 73A and travel along cam path 71A. Similarly, in FIG. 7B, the bone screw 28 has been placed in the hole 26 such that the cam 40 will enter the mouth 73B and travel along cam path 71B. Accordingly, the embodiment of FIGs. 7A and 7B provides the surgeon with two options for locking a bone screw 28 to a plate 20 on opposite sides of the hole.

### Compression

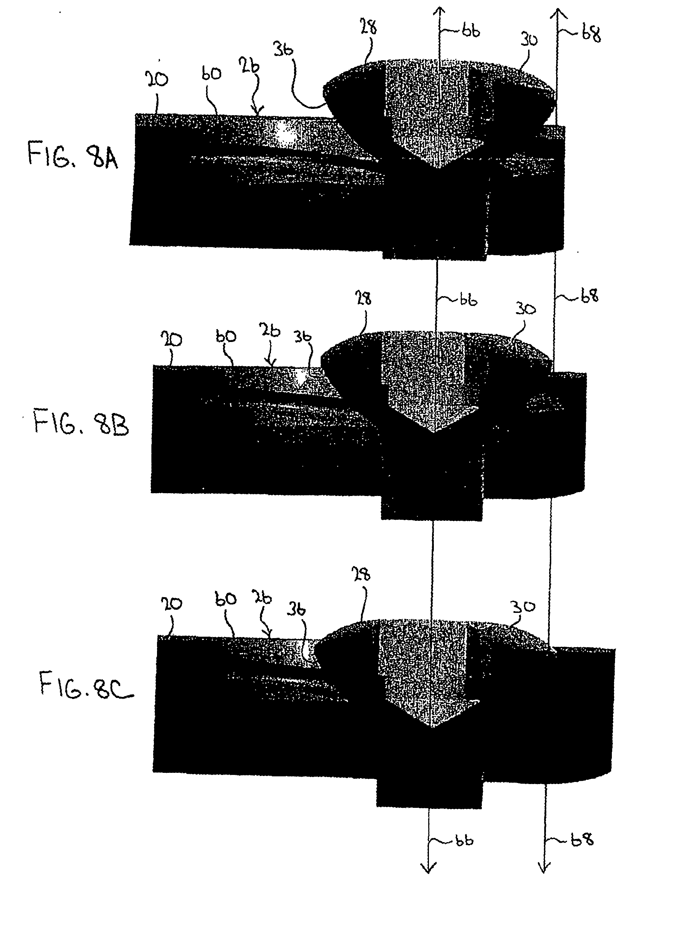

Although a dual cam path elliptical hole 26 is shown in FIGs. 7A and 7B as used for a locking operation, the same hole 26 may also be used for a compression operation. FIGs. 8A-8C show such a compression operation using a dual cam path elliptical hole 26. In FIG. 8A, the bone screw 28 has been positioned along the right side of the hole with the cupped surface 36 of the screw head 30 positioned at the top of the compression ramp 60. As the bone screw 28 is driven into the bone, the cupped surface 36 of the screw head 30 is driven along the compression ramp into more complete engagement with the compression ramp 60. During this action, the bone plate 20 is pulled to the right as a result of the head being forced against the compression ramp. Movement of the bone plate 20 to the right can be seen by the position of the bone plate relative to screw centerline 66 and original hole line 68. The screw centerline 66 shows the centre axis of the screw as it is driven into the bone. The original hole line 68 shows the original position of the right side of the hole before the screw is driven completely into the hole. As shown in FIGs. 8B and 8C, as the screw 28 is driven into the hole, the hole 26 and associated bone plate 20 moves further to the right relative to the screw 28.

In FIG. 8C, the screw head 30 has been driven further into the hole 26, and the cupped surface 36 fully engages the compression ramp 60. In this position, the action of the screw head against the compression ramp 60 has pulled the compression ramp and bone plate further to the right. Accordingly, this action may be used to compress a bone fracture where an opposite end of the plate 20 has been secured to the bone. For example, assuming a fracture is located to the left of the hole 26 shown in FIGs. 8A to 8C, and the bone plate 20 is first secured to the bone portion to the left of the fracture, the action of FIGs. 8A-8C pulls the bone plate and bone to the right, thus compressing the bone at the location of the fracture.

The bone plate described herein may be used as either a locking plate or a compression plate. In particular, a given hole 26 in the bone plate 20 includes both a compression ramp 60 configured to engage the head of a compression screw and a cam path 70 provided on the compression ramp, the cam path 70 configured to engage a cam on the head of a locking screw. With such an arrangement, a surgeon may use a single bone plate 20 as either a locking plate or a compression plate, depending upon the type of screw selected by the surgeon. One of skill in the art will also recognize that the bone plate described herein may also be used as a hybrid plate where some limited compression is offered as well as locking. For example, if a locking screw is used, but the locking screw is located on the edge portion of a hole during insertion, engagement of the cupped lower surface of the locking screw with the compression ramp of the hole will offer compression features before the cam on the locking screw enters the cam path and locks to the bone plate.

### Detachable Cam

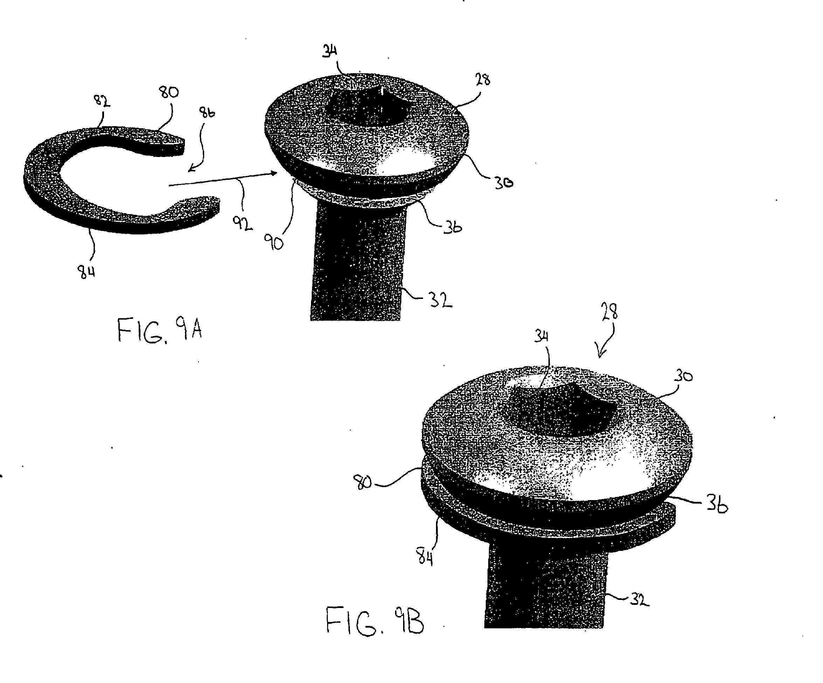

FIGS. 9A to 12C show an embodiment where the bone screw 28 includes a detachable cam 80. As shown in FIG. 9A, the detachable cam 80 is C-shaped and includes an interior perimeter 82 and an exterior perimeter 84. The shape of the detachable cam 80 defines an opening 86 to the interior perimeter 82.

The bone screw 28 configured for use with the detachable cam 80 is shaped similar to other bone screws described herein, and includes a screw head 30 and a threaded screw shaft 32. The screw head includes a slot 34 formed in the top of the head 30 and a cupped lower surface 36. Unlike the other bone screws described herein, the bone screw of FIG. 9A includes a circular groove 90 provided in the cupped lower surface 36.

The circular groove 90 of the screw head 30 is configured to receive the detachable cam 80, as indicated by arrow 92 in FIG. 9A. In particular, the screw head 30 is inserted into the opening in the cam 80, causing the interior perimeter 82 of the cam to engage the circular groove 90 on the screw head. As the cam 80 is inserted onto the screw head 30, the cam slightly deforms as the ends of the C-shaped cam slide across the full diameter of the circular groove 90, and then the cam 80 snaps into place in the groove 90, thus securing the cam 80 within the groove 90, as shown in FIG. 9B. Although the cam 80 is held snugly within the groove 90, the cam 80 may still be rotated around the groove 90 when appropriate force is applied to the cam 80.

The bone screw of FIG. 9A including a detachable cam 80 is configured for use with a bone plate 20 having a hole 26 with a cam path 70 such as that shown in FIGS. 5A and 5B, where the cam path 70 formed in the hole 26 is parallel to the outward facing surface 22 of the bone plate 20.

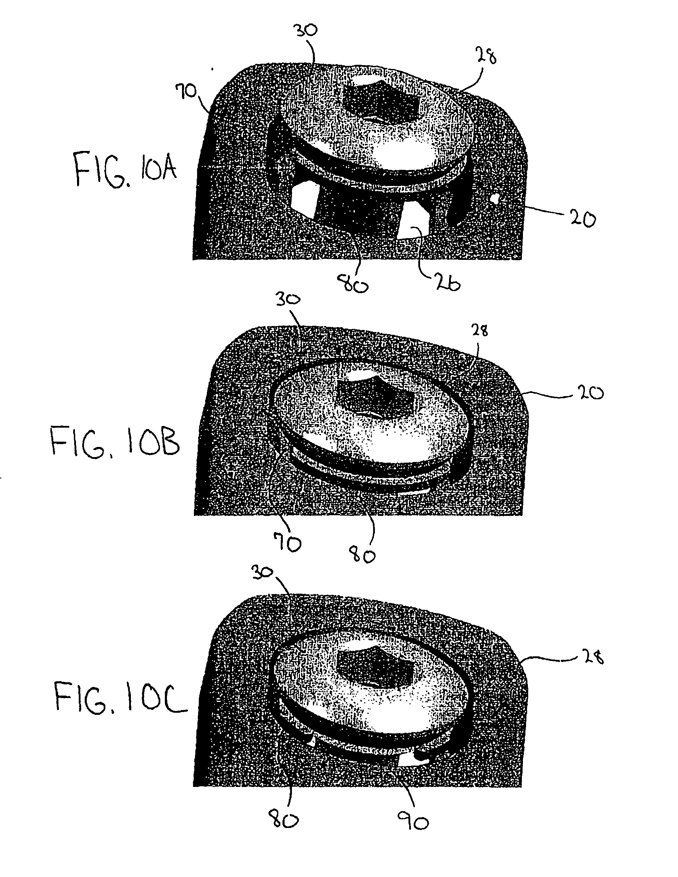

FIGs. 10A to 10C show the bone screw 28 of FIG. 9B being inserted into a hole 26 with a parallel cam path as shown in FIGs. 5A and 5B. Starting with FIG. 10A, the bone screw 28 is shown with the shaft 32 extending the through the hole 26 in the bone plate 20. As the bone screw 28 is rotated, the head 30 moves deeper into the hole, as shown in FIG. 10B. When the cam 80 is adjacent to the cam path 70, the cam 80 may be rotated within the groove 90 in the screw head 30 as shown in FIG. 10C. When the cam 80 is rotated in the screw head 30, the cam 80 fully engages the cam path 70 in the bone plate 20 such that the opening 86 is not positioned within the cam path 70, but is instead centrally located within the hole 26, as shown in FIG. 10C. Rotating the cam 80 in this fashion causes the cam 80 to fully engage the cam path 70 and locks the cam 80 and associated screw 26 in place upon the bone plate 20.

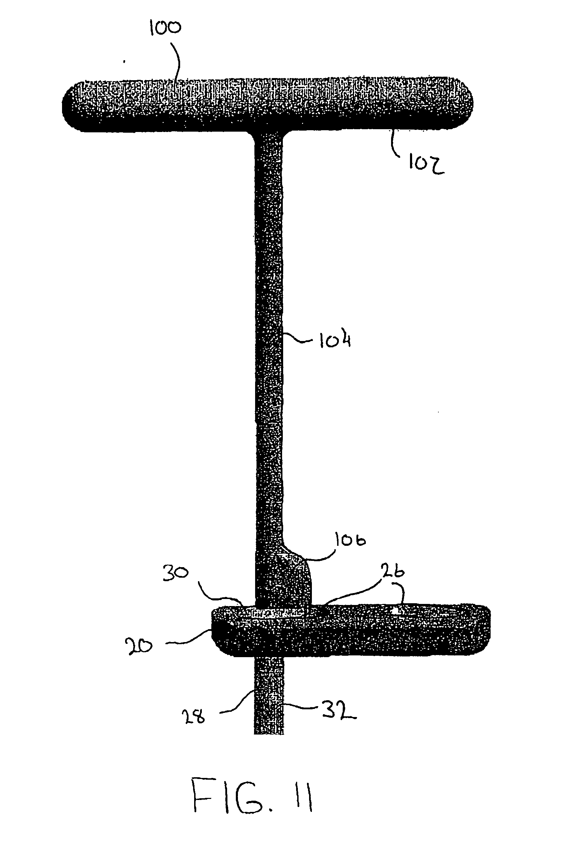

FIG. 11 shows a tool that may be used to rotate the detachable cam 80 within the cam path 70 of the bone plate 20. As shown in FIG. 11, the tool 100 includes a top handle 102 connected to one end of a shaft 104. The opposite end of the shaft 106 is connected to a foot 106. The foot 106 includes a slot finger (not shown) and a cam finger 108. The slot finger is a cylindrical post configured to rotate within the slot 34 on the top of the bone screw 28. The cam finger 108 is configured extend along the cup shaped surface 36 on the head of the bone screw 28 and be positioned within the opening 86 formed by the detachable cam 80. From this position, when the handle 102 of the tool 100 is rotated, the cam finger 108 contacts an end of the cam and forces the cam 80 to rotate within the groove 90 of the screw head and within the cam path 70 of the bone plate 20. Rotation of the cam 80 causes the cam to fully engage the cam path, thus locking the cam 80 and associated screw 26 in place within the bone plate 20.

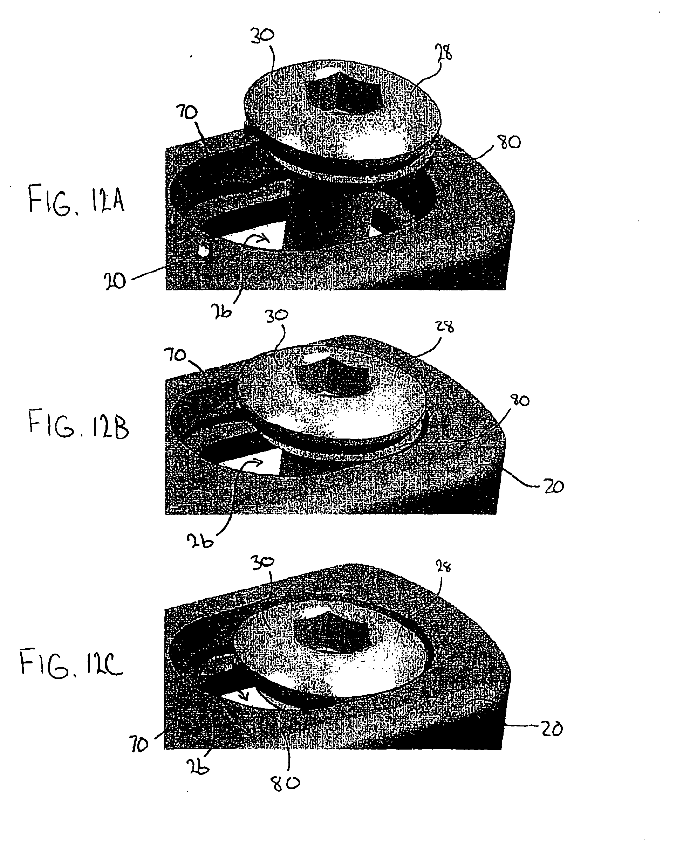

FIGs. 12A to 12C show an alternative embodiment where the detachable cam 80 is self-locking within the bone plate 20. In this embodiment, the cam 80 is comprised of a resilient compressible material, such as ultra high molecular weight polyethylene. Starting with FIG. 12A, the bone screw 28 is shown with the shaft 32 extending the through the hole 26 in the bone plate 20. As the bone screw 28 is rotated, the head 30 moves deeper into the hole, as shown in FIG. 10B. As the head 30 moves even deeper into the hole, the cam 80 is deformed as it is compressed along the compression surface 60 of the hole. When the head 30 becomes fully engage in the hole, the detachable cam 80 snaps into place within the cam path 70, thus locking the cam 80 and associated screw 26 in place within the bone plate 20. In this embodiment, the opening 86 is already properly positioned away from the cam path 70, and there is no need to further rotate the cam 80.

In yet another alternative embodiment, the detachable cam may be inserted into the groove on the head after the screw is fully engaged within the hole of the plate. In this embodiment, the screw may be used to provide compression with the plate, as described previously. After the screw is fully engaged in the hole, the groove 90 is aligned with the parallel cam path 70. The detachable cam is then inserted in the hole and slid into the groove. When the detachable cam is placed in the groove, the cam will also slide into engagement with the cam path. Thereafter, the detachable cam may be rotated to further secure the detachable cam within the cam path. In this and other embodiments as described above with a detachable cam, the bone plate may be used as a compression plate, a locking plate, or a combination thereof.

## Claims

1. A bone plate (20) comprising:
(a) a bone facing surface (24);
(b) an outward facing surface (22) opposite the bone facing surface (24); and
(c) at least one hole (26) extending through the bone plate (20) from the outward facing surface (22) to the bone facing surface (24); and
(d) an arced compression surface (60) provided within the at least one hole (26);
**characterised in** further comprising:
(e) a cam groove (70) forming an indentation in the arced compression surface (60) provided within the at least one hole (26).

2. The bone plate (20) of claim 1, in which the arced compression surface (60) extends 360° within the at least one hole (26).

3. The bone plate (20) of claim 1, in which the cam groove (70) extends less than 360° around the arced compression surface (60).

4. The bone plate (20) of claim 1, in which the cam groove (70) is helical in shape.

5. The bone plate (20) of claim 1, in which the cam groove (70) is flat with respect to the outward facing surface (22) or the bone facing surface (24) of the bone plate (20).

6. The bone plate (20) of claim 1, in which the cam groove (70) is located at an upper edge (50) of the arced compression surface (60).

7. The bone plate (20) of claim 1, in which the cam groove (70) is located at a lower edge (54) of the arced compression surface (60).

8. The bone plate (20) of claim 1, in which the at least one hole (26) is circular or elliptical.

9. The bone plate (20) of claim 1, in which the cam groove (70) is a first cam groove (73a) and in which the bone plate (20) has a second cam groove (73b) formed within the at least one hole (26).

10. A bone screw (28) configured for insertion into a bone plate (20), the bone screw (28) comprising:
(a) a threaded shaft (32); and
(b) a head (30) connected to the shaft (32), the head (30) comprising a top face and an arced compression surface (36), the arced compression surface (36) extending upwardly and outwardly from the shaft (32);
**characterised in** further comprising:
(c) a cam (40) connected to the head (30), the cam (40) protruding from the arced compression surface (36) and extending one revolution or less around the arced compression surface (36).

11. The bone screw (28) of claim 10, in which the cam (40) extends around arced compression surface (36) in a helical fashion.

12. The bone screw (28) of claim 10, in which the cam (40) is releasibly connected to the head (30).

13. The bone screw (28) of claim 12, in which the head (30) comprises a cam groove (90) and the cam (80) is retained within the cam groove (90).

14. The bone screw (28) of claim 13, in which the cam (80) comprises a C-shaped member designed and dimensioned to fit within the cam groove (90).

15. A bone plate assembly comprising a bone plate (20) according to claim 1 and a bone screw (28) according to claim 10.

16. The bone plate assembly of claim 15, wherein:
the bone plate (20) is elongated and defines a central longitudinal axis (21); and
the arced compression surface (60) is provided on two directly opposing sides of the at least one hole (26) along the central longitudinal axis (21).

17. The bone plate assembly of claim 16, in which the head (30) of the bone screw (28) is configured to engage the arced compression surface (60) such that the bone plate (20) acts as a compression plate.

18. The bone plate assembly of claim 16, in which the head (30) of the bone screw (28) comprises at least one cam (40) attached to the head (30) , in which the cam (40) is configured to engage the cam groove (70) such that the bone plate (20) acts as a locking plate.

19. The bone plate assembly of claim 18, in which the cam (90) is releasibly attached to the head (30) of the bone screw (28).

20. The bone plate assembly of claim 18, in which the cam (90) is configured to rotate upon the head (30).

21. The bone plate assembly of claim 20, which includes a tool (100) configured to engage the bone screw (28) and rotate the cam (40) upon the head (30).

22. The bone plate assembly of claim 18, in which the cam (40) is configured to snap into the cam groove (70).

23. The bone plate assembly of claim 18, in which the cam (40) is configured to lock into the cam groove (70) after at least 90° of revolution of the bone screw (28) following entry of the cam (40) into the cam groove (70).

## Patentansprüche

1. Knochenplatte (20), die aufweist:
(a) eine zum Knochen weisende Fläche (24);
(b) eine nach außen weisende Fläche (22), die der zum Knochen weisenden Fläche (24) gegenüberliegt; und
(c) wenigstens ein Loch (26), das sich durch die Knochenplatte (20) von der nach außen weisenden Fläche (22) zu der zum Knochen weisenden Fläche (24) erstreckt; und
(d) eine bogenartige Druckfläche (60), die innerhalb des wenigstes einen Lochs (26) vorgesehen ist;
**dadurch gekennzeichnet, dass** sie weiter aufweist:
(e) eine Führungsnut (70), die in einer Vertiefung der bogenartigen Druckfläche (60), die innerhalb des wenigstens einen Loches (26) vorgesehen ist, gebildet ist.

2. Knochenplatte (20) nach Anspruch 1, bei der sich die bogenartige Druckfläche (60) innerhalb des wenigstens einen Loches (26) über 360° erstreckt.

3. Knochenplatte (20) nach Anspruch 1, bei der sich die Führungsnut (70) über weniger als 360° um die bogenartige Druckfläche (60) erstreckt.

4. Knochenplatte (20) nach Anspruch 1, bei der die Führungsnut (70) in der Form wendelartig ist.

5. Knochenplatte (20) nach Anspruch 1, bei der die Führungsnut (70) in Bezug auf die nach außen weisende Fläche (22) oder die zum Knochen weisende Fläche (24) der Knochenplatte (20) eben ist.

6. Knochenplatte (20) nach Anspruch 1, bei der sich die Führungsnut (70) an einer oberen Kante (50) der bogenartigen Druckfläche (60) befindet.

7. Knochenplatte (20) nach Anspruch 1, bei der sich die Führungsnut (70) an einer unteren Kante (54) der bogenartigen Druckfläche (60) befindet.

8. Knochenplatte (20) nach Anspruch 1, bei der das wenigstens eine Loch (26) kreisförmig oder elliptisch ist.

9. Knochenplatte (20) nach Anspruch 1, bei der die Führungsnut (70) eine erste Führungsnut (73a) ist und wobei die Knochenplatte (20) eine zweite Führungsnut (73b) hat, die innerhalb des wenigstens einen Loches (26) gebildet ist.

10. Knochenschraube (28), die zum Einsetzen in eine Knochenplatte (20) ausgelegt ist, wobei die Knochenschraube (28) aufweist:
(a) einen mit Gewinde versehenen Schaft (32); und
(b) einen Kopf (30), der mit dem Schaft (32) verbunden ist, wobei der Kopf (30) eine obere Fläche und eine bogenartige Druckfläche (36) aufweist, wobei sich die bogenartige Druckfläche (36) von dem Schaft (32) nach oben und außen erstreckt;
**dadurch gekennzeichnet, dass** sie weiter aufweist:
(c) einen Nocken (40), der mit dem Kopf (30) verbunden ist, wobei der Nocken (40) von der bogenartigen Druckfläche (36) hervorsteht und sich eine Umdrehung oder weniger um die bogenartige Druckfläche (36) erstreckt.

11. Knochenschraube (28) nach Anspruch 10, bei der sich der Nocken (40) in einer wendelartigen Weise um die bogenartige Druckfläche (36) erstreckt.

12. Knochenschraube (28) nach Anspruch 10, bei der der Nocken (40) lösbar mit dem Kopf (30) verbunden ist.

13. Knochenschraube (28) nach Anspruch 12, bei der der Kopf (30) eine Führungsnut (90) aufweist und der Nocken (80) in der Führungsnut (90) gehalten wird.

14. Knochenschraube (28) nach Anspruch 13, bei der der Nocken (80) ein C-förmiges Element aufweist, das so gestaltet und gemessen ist, dass es in die Führungsnut (90) passt.

15. Knochenplattenanordnung, die eine Knochenplatte (20) nach Anspruch 1 und eine Knochenschraube (28) nach Anspruch 10 aufweist.

16. Knochenplattenanordnung nach Anspruch 15, bei der:
die Knochenplatte (20) länglich ist und eine mittlere Längsachse (21) definiert;
die bogenartige Druckfläche (60) auf zwei direkt gegenüberliegenden Seiten des wenigstens einen Loches (26) entlang der mittleren Längsachse (21) vorgesehen ist.

17. Knochenplattenanordnung nach Anspruch 16, bei der der Kopf (30) der Knochenschraube (28) so ausgelegt ist, dass er an der bogenartigen Druckfläche (60) angreift, so dass die Knochenplatte (20) als eine Druckplatte wirkt.

18. Knochenplattenanordnung nach Anspruch 16, bei der der Kopf (30) der Knochenschraube (28) wenigstens einen Nocken (40) aufweist, der an dem Kopf (30) befestigt ist, wobei der Nocken (40) so ausgelegt ist, dass er in die Führungsnut (70) eingreift, so dass die Knochenplatte (20) als eine verriegelnde Platte wirkt.

19. Knochenplattenanordnung nach Anspruch 18, bei der der Nocken (90) lösbar an dem Kopf (30) der Knochenschraube (28) befestigt ist.

20. Knochenplattenanordnung nach Anspruch 18, bei der der Nocken (90) so ausgelegt ist, dass er sich um den Kopf (30) dreht.

21. Knochenplattenanordnung nach Anspruch 20, die ein Werkzeug (100) umfasst, das so ausgelegt ist, dass es die Knochenschraube (28) greift und den Nocken (40) um den Kopf (30) dreht.

22. Knochenplattenanordnung nach Anspruch 18, bei der der Nocken (40) so ausgelegt ist, dass er in die Führungsnut (70) einrastet.

23. Knochenplattenanordnung nach Anspruch 18, bei der der Nocken (40) so ausgelegt ist, dass er nach wenigstens 90° Drehung der Knochenschraube (28) nach dem Einsetzen des Nockens (40) in die Führungsnut (70) sperrt.

## Revendications

1. Lame osseuse (20) comprenant :
(a) une surface orientée vers l'os (24) ;
(b) une surface orientée vers l'extérieur (22) opposée à la surface orientée vers l'os (24) ; et
(c) au moins un trou (26) s'étendant à travers la lame osseuse (20) depuis la surface orientée vers l'extérieur (22) à la surface orientée vers l'os (24) ; et
(d) une surface de compression arquée (60) fournie dans ledit au moins un trou (26) ;
**caractérisée en ce qu'**il comprend en outre :
(e) une cannelure de came (70) formant une indentation dans la surface de compression arquée (60) ménagée dans ledit au moins un trou (26).

2. Lame osseuse (20) selon la revendication 1, dans laquelle la surface de compression arquée (60) s'étend sur 360° à l'intérieur du au moins un trou (26).

3. Lame osseuse (20) selon la revendication 1, dans laquelle la cannelure de came (70) s'étend sur moins de 360° autour de la surface de compression arquée (60).

4. Lame osseuse (20) selon la revendication 1, dans laquelle la cannelure de came (70) est de forme hélicoïdale.

5. Lame osseuse (20) selon la revendication 1, dans laquelle la cannelure de came (70) est plane relativement à la surface orientée vers l'extérieur (22) ou la surface orientée vers l'os (24) de la lame osseuse (20).

6. Lame osseuse (20) selon la revendication 1, dans laquelle la cannelure de came (70) est située à un bord supérieur (50) de la surface de compression arquée (60).

7. Lame osseuse (20) selon la revendication 1, dans laquelle la cannelure de came (70) est située à un bord extérieur (54) de la surface de compression arquée (60).

8. Lame osseuse (20) selon la revendication 1, dans laquelle ledit au moins un trou (26) est circulaire ou elliptique.

9. Lame osseuse (20) selon la revendication 1, dans laquelle la cannelure de came (70) est une première cannelure de came (73a) et dans lequel la lame osseuse (20) a une seconde cannelure de came (73b) formée dans ledit au moins un trou (26).

10. Vis osseuse (28) configurée pour l'insertion dans une lame osseuse (20), la vis osseuse (28) comprenant :
(a) un arbre fileté (32) ; et
(b) une tête (30) connectée à l'arbre (32), la tête (30) comprenant une face supérieure et une surface de compression arquée (36), la surface de compression arquée (36) s'étendant vers le haut et vers l'extérieur de l'arbre (32) ;
**caractérisée en ce qu'**elle comprend en outre :
(c) une came (40) connectée à la tête (30), la came (40) faisant saillie depuis la surface de compression arquée (36) et s'étendant sur une révolution ou moins autour de la surface de compression arquée (36).

11. Vis osseuse (28) selon la revendication 10, dans laquelle la came (40) s'étend autour de la surface de compression arquée (36) de manière hélicoïdale

12. Vis osseuse (28) selon la revendication 10, dans laquelle la came (40) est raccordée de manière amovible à la tête (30).

13. Vis osseuse (28) selon la revendication 12, dans laquelle la tête (30) comprend une cannelure de came (90) et la came (80) est retenue dans la cannelure de came (90).

14. Vis osseuse (28) selon la revendication 13, dans laquelle la came (80) comprend un élément en forme de C, conçu et dimensionné pour s'adapter dans la cannelure de came (90).

15. Ensemble de lame osseuse comprenant une lame osseuse (20) selon la revendication 1 et une vis osseuse (28) selon la revendication 10.

16. Ensemble de lame osseuse selon la revendication 15, dans lequel :
→ la lame osseuse (20) est allongée et définit un axe longitudinal central (21) ; et
→ la surface de compression arquée (60) est ménagée sur deux côtés directement opposés dudit au moins un trou (26) le long de l'axe longitudinal central (21).

17. Ensemble de lame osseuse selon la revendication 16, dans lequel la tête (30) de la vis osseuse (28) est configurée pour mettre en prise la surface de compression arquée (60), de sorte que la lame osseuse (20) agisse comme une plaque de compression.

18. Ensemble de lame osseuse selon la revendication 16, dans lequel la tête (30) de la vis osseuse (28) comprend au moins une came (40) fixée à la tête (30), dans laquelle la came (40) est configurée pour mettre en prise la cannelure de came (70), de sorte que la lame osseuse (20) agisse comme une plaque de verrouillage.

19. Ensemble de lame osseuse selon la revendication 18, dans lequel la came (90) est fixée de manière amovible à la tête (30) de la vis osseuse (28).

20. Ensemble de lame osseuse selon la revendication 18, dans lequel la came (90) est configurée pour tourner sur la tête (30).

21. Ensemble de lame osseuse selon la revendication 20, qui comprend un outil (100) configuré pour mettre en prise la vis osseuse (28) et tourner la came (40) sur la tête (30).

22. Ensemble de lame osseuse selon la revendication 18, dans lequel la came (40) est configurée pour s'insérer dans la cannelure de came (70).

23. Ensemble de lame osseuse selon la revendication 18, dans lequel la came (40) est configurée pour verrouiller dans la cannelure de came (70) après au moins 90° de révolution de la vis osseuse (28) après l'entrée de la came (40) dans la cannelure de came (70).
